# EUROPEAN PATENT SPECIFICATION

(11) **EP 4 612 521 B1**
(45) Date of publication and mention of the grant of the patent: **14.10.2026**
(21) Application number: 23799027.0
(22) Date of filing: 30.10.2023
(51) Int. Cl.: G01S 15/89, G01S 7/52, A61B 8/08, A61B 8/00

(54) **SYSTEM AND METHOD FOR FORMING AN IMAGE OF ULTRASOUND REFLECTIVITY**
SYSTEM UND VERFAHREN ZUR ERZEUGUNG EINES BILDES VON ULTRASCHALLREFLEKTIVITÄT
SYSTÈME ET PROCÉDÉ DE FORMATION D'UNE IMAGE DE RÉFLECTIVITÉ ULTRASONORE

(30) Priority: 31.10.2022 EP 22204798
(43) Date of publication of application: 10.09.2025
(73) Proprietor: Nederlandse Organisatie voor toegepast- natuurwetenschappelijk onderzoek TNO, 2595 DA 's-Gravenhage (NL)
(72) Inventor: VOLKER, Arno Willem Frederik, 2595 DA 's-Gravenhage (NL); VAN NEER, Paul Louis Maria Joseph, 2595 DA 's-Gravenhage (NL); VROLIJK, Jan-Willem, 2595 DA 's-Gravenhage (NL)
(74) Representative: V.O.
(86) International application number: PCT/NL2023/050569
(87) International publication number: WO 2024/096733

(56) References cited:
- EP-B1- 3 347 736
- WO-A1-2014/136461
- YEN J T ET AL: "Real-time rectilinear volumetric imaging", IEEE TRANSACTIONS ON ULTRASONICS, FERROELECTRICS, AND FREQUENCY CONTROL, IEEE, USA, vol. 49, no. 1, 1 January 2002 (2002-01-01), pages 114 - 124, XP011368221, ISSN: 0885-3010, DOI: 10.1109/58.981389

## Description

### Field

The invention relates to ultrasound imaging using an array of ultrasound transducers.

### Background

A large area two dimensional array of Piezo-electric columns on a substrate for use as ultrasound transducers has been described in a poster presentation by van Neer et al. at the IEEE IUS 2021, titled "Flexible ultrasound transducer array concept scalable to large areas: first 128 element 7 MHz linear array". The technology for manufacturing such arrays has been described in a poster presentation by van Peters et al. at the IEEE IUS 2021 titled "Technology for Large Area and Flexible ultrasound patches. The array comprises uniform pillars of small size (e.g. 40x40x80 micrometer) with successive pillars at a small pitch (e.g. 60 micrometer) and the array may have a large size (e.g. 150x150 mm with more than six million pillars). The array can be used to form a three dimensional image or one or more two dimensional images of reflection from positions in an object space with distance in the direction perpendicular to the array. A flexible substrate can be used that makes the array flexible. Due to the flexibility of the substrate, the array can be used as a wearable device on the body of a person. This makes it possible to obtain images for medical purposes during normal life activities of a patient.

However use of a large array requires considerable time to perform the measurements and computations needed for imaging. Unlike reading data from a memory array or an optical image from an optical sensor array, sensing the signal from individual receiving elements in the ultrasound reception array must be performed as a function of time. This may need to be repeated successively for many different signals to collect sufficient information for imaging, and digitized signals from many individual receiving elements must be combined to obtain high three dimensional resolution.

The considerable amount of time needed to obtain an image limits its usefulness. For example, it cannot be expected that a person will remain motionless during the measurements when the device is used as a wearable imaging device. In addition, a large processing time may be needed to perform the necessary signal processing. This could be reduced by using more powerful processing circuitry, but this may hinder wearable use.
The time needed for such measurements can be reduced to some extent by receiving ultrasound from array elements from rows of locations in the array in parallel and/or transmitting from different locations in parallel. With more complex wiring than for a simple array the amount of parallelism could be increased further, but it is desirable to avoid too much complexity.

Another way of reducing the time needed to obtain an image is to use spatial sub-sampling, e.g. by processing only signals received at a subset of locations in the array. An array with a pitch of 50-500 micrometer can produce a much higher resolution than needed for many purposes, such as obtaining a large scale medical image, so that the loss of resolution due to sub-sampling may not be a problem. However, spatial sub-sampling can create image artifacts such as aliasing, or a need to suppress such artifacts at the expense of resolution .

EP 3 347 736 B1 describes a system of combined phased-array and Fresnel zone plate beamforming employing delay-corrected Fresnel subapertures.

WO 2014/136461 A1 describes an ultrasound sensor element for use in a two-dimensional array of ultrasound sensor elements.

Publication "Real-time rectilinear volumetric imaging" (Yen J. T. et al.), IEEE TRANSACTIONS ON ULTRASONICS, FERROELECTRICS, AND FREQUENCY CONTROL, IEEE, USA, vol. 49, no. 1, 1 January 2002 (2002-01-01), pages 114-124, ISSN: 0885-3010, DOI: 10.1109/58.981389, describes a rectilinear volumetric scanner, that utilizing a sparse 94x94 Mills cross array, to overcome the limitations of current pyramidal scanners for non-cardiac applications.

### Summary of the invention

Among others, it is an object to provide for efficient ultrasound imaging using an ultrasound transducer array with a large number of transducers.

A method according to claim 1 is provided for forming an object image of ultrasound reflectivity in an object space, using an array of rows and columns of ultrasound transducers that define a sensing surface adjacent the object space. In the method, object image values for each position in the image are computed by summing cross-point based image values for the position determined for a plurality of cross-points locations that are each associated with a respective combination of a row and column in the array. The cross-point based image value for a position in the image is determined using transmission from sub-sampled locations from a range of positions that lie no further from the column of the respective cross point location than from the row of the respective cross-point location preferably in the column of the respective cross point location), to provide for synthetic directional sensitivity at least in the column direction of the array, and reception or resulting reflection from sub-sampled locations from a range of positions in a row, to provide for synthetic directional sensitivity in the row direction of the array. At least the ranges for neighboring cross-points overlap with each other, e.g. so that the range is twice, three times, four times or more as large as the distance between the cross-points. The sampling locations used for different cross-points in the overlap lie at least partly interspersed with each other.

The cross-point based image values computed for different positions in object space may constitute a complete image as a function of those positions. But although cross-point images are used that are images of the same (part of) object space, the cross-point based object image determined for different cross-point locations are made different because different sub-sampling in the ranges in both the row direction and the column direction are used for different cross-point based images. The subsampling may be quite deep, e.g. using only one in ten positions along the row and offset from the row on average. Sub-sampling based on random selections may be used.

Deep sub-sampling saves computation time, but creates sampling artifacts. These sampling artifacts are reduced by using sums of cross-point based image values, in combination with at least partly interspersed, different sampling selections from overlapping ranges for different cross-points. In this way, the sampling artifacts in different cross-point based images at least partly compensate each other in the summed image.

Preferably, interspersed sub-sampling locations are selected to avoid coincidence between sub-sampling locations and coincidence between locations at sub-sampled offsets from the row (e.g. between sub-sampling locations in the column) for nearby cross-point locations. But even with partial coincidence, the sub-sampling artifacts in different cross-point based images at least partly compensate each other in the summed image. With deep sub-sampling it is easy to avoid such coincidences.

At least partially interspersed sampling locations can be realized by independent random selection of the sampling locations for different cross point locations. This may result in coincidence of some of the sampling locations for different cross point locations, but with deep sub-sampling this will occur infrequently.

In an embodiment, the first selection and the second selection are a-periodic selections. This may be used to further reduce strongly visible subsampling artifacts. In a further embodiment, the values of distances between successive ones of the first sampling locations and/or between successive ones of the second sampling locations lie distributed over a predetermined distance value range that contains a plurality of distance values. Thus, strong local sampling density variations can be prevented. The distance values may be distributed similar to a pseudo random selection from the predetermined distance value range. In a further embodiment, a lower limit of the predetermined distance value range is at most half an average of the values of the distances.

In an embodiment, the cross points are located on a two-dimensional periodic grid of locations, with a period smaller than the first and second ranges. This provides for uniform cross point image determination. But in other embodiments, a-periodically located cross points may be used to reduce artifacts.

In an embodiment, average sub-sample ratios of the first and second selections are at most half a density of the cross points along the column and/or row of the respective cross point location. Thus, deep subsampling may be used. Normally, this would cause strong artifacts, but the use of sums of cross-point images based on differing selections from overlapping ranges, makes it possible to use such deep sub-sampling with less artifacts.

In an embodiment, the summing of the cross-point based image values for the position may be a weighted summing. Thus for example, less or no weight may be given to cross-point images for cross-points that lie farther away from the object space position for which an image value is computed. This can be used to reduce noise.

In a further embodiment, the weighted summing defines a window of cross-point locations of which the first and/or second ranges all overlap with the first and/or second range of at least one of the cross-point locations in the window. Thus, the artifact reduction can be optimized. The least one of the cross-point locations in the window may be the cross-point location that lies closest to the object space position for which an image value is computed. Preferably, no weight is given to cross-point locations with ranges that do not overlap with the ranges of the least one of the cross-point locations. Thus, noise can be reduced.

### Brief description of the drawing

These and other objects and advantageous aspects will be illustrated in a detailed description of exemplary embodiments with reference to the following figures.
Figure 1 shows a top view of an ultrasound imaging device
Figure 2a show an imaging system
Figure 2b shows a signal flow model
Figure 2c shows a matrix with a reduced number of elements
Figure 3 shows a linear excitation/reception pattern
Figure 4 shows a side view a wearable ultrasound imaging device
Figure 5 shows a circuit diagram of an array element
Figure 6 shows a flow chart of imaging
Figure 7 shows a flow chart of image computation
Figure 8 illustrates an embodiment with reception with multiple rows

### Detailed description of exemplary embodiments

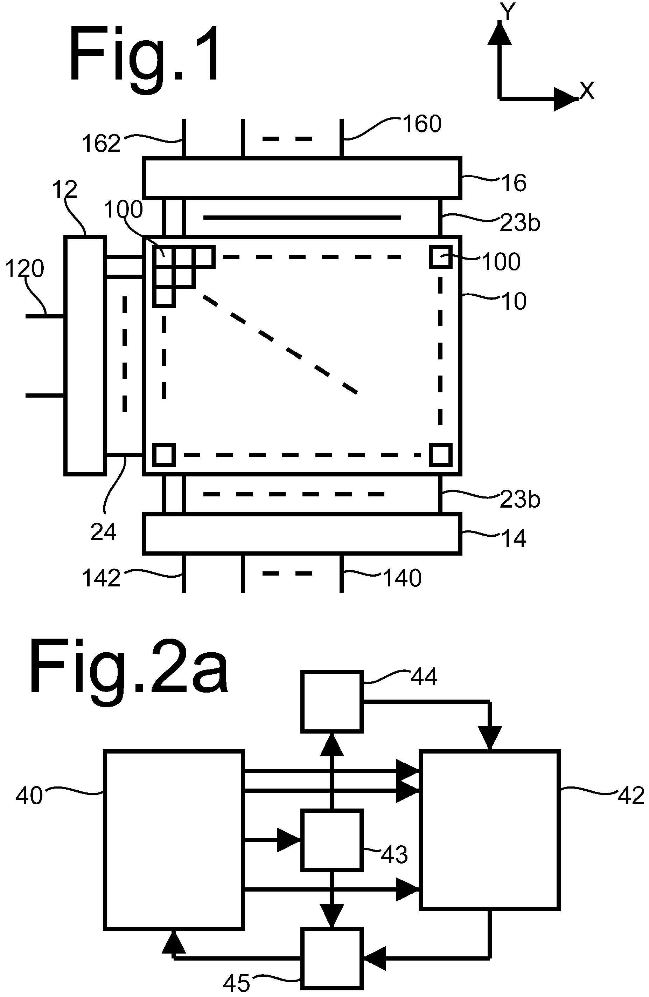

Figure 1 shows an example of an ultrasound imaging device with two-dimensional array 10 of ultrasound transducer elements, with the array extending laterally in x and y directions, as viewed from a position in the z-direction perpendicular to array 10. Imaging using the ultrasound imaging device involves both ultrasound transmission and ultrasound reception by the array. Each ultrasound transducer element may be an ultrasound receiver, an ultrasound transmitter or both. It should be noted that although the layout of the array may look like that of an optical image sensor, ultrasound imaging is more complex than optical imaging: in addition to the fact that the array is used both for transmission and reception, the signals at each array element must be processed as a time dependent ultrasound signal.

Array 10 is located on a substrate, which may be a flexible substrate. Array 10 may have a rectangular shape, with sides of a hundred of millimeters or hundreds of millimeters more long. At the same time, array elements may be provided at a much smaller pitch of less than a hundred micrometer (e.g. sixty micrometers), resulting in thousands of ultrasound transducer elements along the sides of the array and millions of ultrasound transducer elements in the array.

The ultrasound imaging device may be used as a wearable ultrasound imaging device, e.g. to be worn on a human or animal body, to capture 3D images of ultrasound reflectivity in a range of depths below the area on the body where the device is worn. The device can be used to obtain an image over the entire area, or an image over a much smaller sub-area, or both. For the purpose of obtaining an overview image over the entire area, the number of array elements is redundantly large. In principle, deep subsampling, e.g. at a rate of one in ten in both the x and y direction may suffice to overview image.

Array 10 comprises rows and columns of uniformly sized array elements 100. The ultrasound imaging device is designed to generate ultrasound signals from array elements 10 at selectable locations in the array, or from selectable groups of such locations and to output electric signals excited by reception of ultrasound at selectable locations in the array, or at selectable groups of such locations.

A row selection circuit 12 and first and second column access circuits 14, 16 are located adjacent array 10 on the substrate. In the illustrated embodiment, the first column access circuit 14 may be used for signals received from the array elements, and second column access circuit 16 may be used for supplying signals for transmission by array elements. Although an embodiment with two column access circuits 14, 16 is shown for the sake of illustration, it should be noted that one column access circuit combining the functions of first and second column access circuits 14, 16 may suffice, for both transmission and reception of signals.

Array 10 may comprise electrically conductive row lines 24 coupled to row selection circuit 12, and electrically conductive column lines 23b coupled to column access circuits 14, 16. Row selection circuit 12 has row address inputs 120 and outputs coupled to row lines 24. Each row line 24 connects row selection circuit 12 to selection inputs of array elements in a respective row of array 10. Column access circuits 14, 16 have inputs and outputs coupled to column lines 23b respectively. Column access circuits 14, 16 have column address inputs 140, 160 and signal inputs 162 and signal outputs 142 for ultrasound excitation signals and ultrasound detection signals. Each column line 23b connects column selection circuits 14, 16 to signal connections of array elements in a respective column of array 10.

Figure 2a shows an imaging system comprising a processing system 40, a wearable ultrasound imaging device 42 with an array such as described by means of figure 1, a timing circuit 43, a signal generator 44 and a detection circuit 45. Together with row selection circuit 12 and second column access circuit 16, signal generator 44 forms an excitation circuit that is configured to cause ultrasound emission from selectable first ultrasound transducers in the array. Together with row selection circuit 12 and first column access circuit 14, detection circuit 45 forms the reception circuit configured to receive ultrasound measurement signals from selectable second ultrasound transducers in the array. As may be noted, the excitation circuit and the reception circuit may share some components.

Processing system 40 has outputs coupled to address inputs of wearable ultrasound imaging device 42, for supplying column addresses to the column access circuits of wearable ultrasound imaging device 42 and for supplying row addresses to the row selection circuit of wearable ultrasound imaging device 42.

Furthermore, processing system 40 has a control output coupled to timing circuit 43. Timing circuit 43 has an output coupled to signal generator 44 and detection circuit 45 .Generator 44 has an output coupled to wearable ultrasound imaging device 42 for supplying a transmission signal to the signal input of the first column access circuit of wearable ultrasound imaging device 42. Detection circuit 45 has an input for receiving a reception signal from the first column access circuit of wearable ultrasound imaging device 42.

In operation, signal generator 44 generates signals for transmission by array elements in rows and columns of the array that are selected by processing system 40. Detection circuit 45 detects signals received by array elements in rows and columns of the array that are selected by processing system 40. Timing circuit 43 controls the time of generation of the signals for transmission and the time reference for detection. Detection circuit 45 may be configured to sample and digitize received signals from the array elements, preferably sampling and digitizing received signals from a plurality of columns from a same row simultaneously, the time reference defining the relation between the sampling times and the generation of the transmitted signal. Detection circuit 45 has an output coupled to processing circuit 40, for supplying the sampled digitized signals.

Although the input of detection circuit 45 is illustrated as a single line, it should be appreciated that the line to detection circuit 45 may represent a single signal input, for use to receive a signal from an individual selected column line or a plurality of signal inputs, for receive and detected signals from a plurality of selected column lines in parallel. In an embodiment detection circuit 45 has a multi-line input for receiving reception signals from all columns of array 10 in parallel. In another embodiment detection circuit 45 has a multi-line input for receiving reception signals from a selectable sub-set of the columns of array 10 in parallel.

Similarly, although the output of signal generator 44 is illustrated as a single line which may represent a single signal output, for use to supply a signal to an individual selected column line, in some embodiments this line may represent a plurality of signal outputs.

Processing system 40 is programmed to control operation of the imaging system. In operation under control of processing system 40, processing system 40 computes images of ultrasound reflectivity in an object space, that is, a space containing points at a range of distances in the z-direction from the xy-plane of array 10, which will be more generally referred to as the sensing surface. The reflections may result from within an object located in the object space. When wearable ultrasound imaging device 42 is worn on a human or animal body, the reflections result from within the body at the location where the device is worn to some depth within the body.

Processing system 40 may be programmed to compute a three dimensional image of reflectivity, or a two dimensional image of reflectivity in a cross-section of the object with a virtual plane in the object space or with a curved virtual surface in the object space. Positions in the object space will be denoted by "r"=(rx, ry, rz) and locations on the sensing surface will be denoted by p=(px, py), which in terms of object space coordinates corresponds to p=(px, py, 0). As used herein, an "image" of reflectivity is a set of image values (or their Fourier transform) that corresponds to reflectivity at positions in the object space in the sense that the set of image values provides information about position dependence of the actual reflectivity in the object space, not necessarily in terms of actual reflectivity values or without any distortion: the amplitude scale or spatial scale may differ from the actual reflectivity, and deviations due to the approximate nature of image computation are not excluded.

### Signal flow for image computation

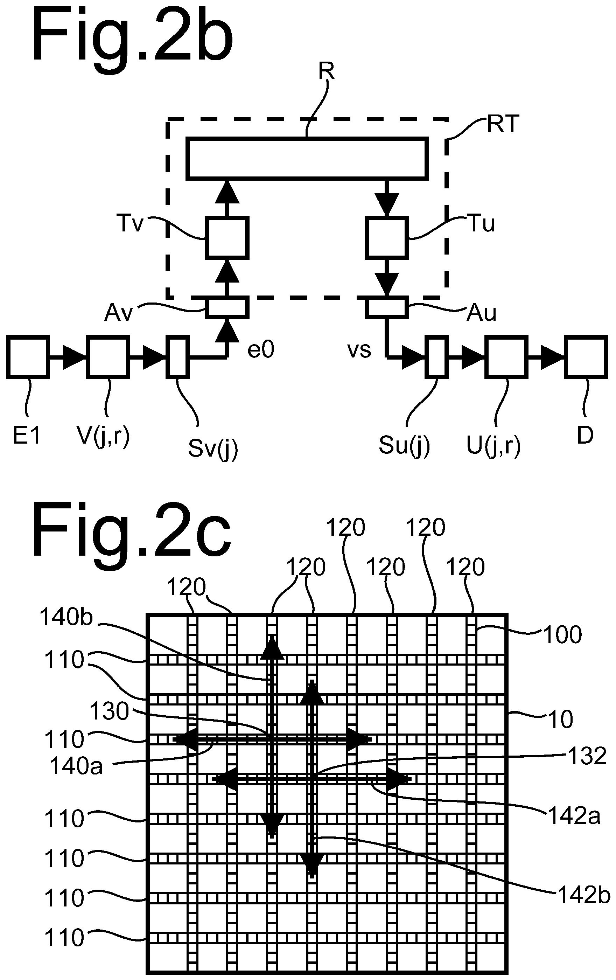

Figure 2b shows a signal flow model for determination of an image Im(r) from reflectivity from the object space. The determination of the image involves transmitting signals from array elements 100, receiving reflected signals at the array elements and computing resulting image values Im(r) from the received signals.

The excitation circuit is represented in terms of a signal generator E1, a vector response filter V(j,r) and spatial sampling functions Sv(j), Av. The reception circuit is represented in terms of spatial sampling functions Au, Su(j), a vector response filter U(j,r) and a signal detector D.

In figure 2b, Av, Au represent sampling due to the fact that array 10 comprises discrete array elements. Sv, Su represent sub-sampling among these elements, which will be discussed later. Av, Au may be absorbed in Sv and Su. Initially ignoring Sv and Su, the transmitted signals for obtaining Im(r) can be expressed as $e 0 = V \left(r\right) * s$

Herein s is a signal that controls the timing and can be represented as a time domain signal or a Fourier transform domain signal (this dependence is left implicit), "*" denotes convolution in the time domain or multiplication in the Fourier transform domain. "s" is used to enable distinction of different reception time delays, and may be a pulse signal for example. Formally, V(r) is a vector with vector components for each of the transmitter elements in array 10, wherein each component may representing a time dependent response function applied to s to obtain a signal for a respective array element, dependent on the position "r" for which the image value Im(r) is determined. "s" may be chosen dependent on the position "r" as well, but this is not necessary, and therefore the dependence on "r", if any, will not be shown.

Furthermore, the determination of Im(r) involves a similar convolution or product and scalar product of a vector D*U(r) with a measured vector signal vs. $Im \left(r\right) = D \left(r\right) * U \left(r\right) * vs$

Herein vs formally is a vector with components for, in principle, each of the array elements, representing the received signals in the time domain or Fourier transform domain. U(r) is a vector of time dependent response functions. D is an operation applied to the scalar product U(r)*vs to select distance, by distinguishing signal values for different time delays. In an embodiment wherein s is a pulse signal, D may correspond to selecting the value of U(r)*vs with the predetermined time delay. When signal e is more complex, D may be correspondingly more complex. "D" may depend on the position "r" for which the image value Im(r) is determined. But the dependence on "r", if any, will not be shown.

The reflectivity in the object space relates the received signal vs to the transmitted signal $vs = R ′ * e 0$

Herein, R' is a matrix with rows and columns, the number of rows and columns both being equal to the number of transmitter elements and receiver elements in array 10 respectively (i.e. not equal to the number of rows or columns in array 10) and "*" denotes convolution in the time domain or multiplication in the Fourier transform domain. The matrix R' can be modeled as
R'=Integral over r' and r" in the object space of Tu(r')*R(r'-r") *Tv (r")

Herein r' and r" are positions in object space, R represents the local reflectivity in the object space (a continuous function), and Tv and Tu are vectors that represent ultrasound propagation from and to locations on the array via the positions in object space respectively. Tv and Tu are functions of position r in object space and travel time. Formally, the products of Tu and Tv with R involve convolutions over object space (or products in Fourier space). But when the reflectivity R may be assumed to be local and instantaneous, the product with Tv will become a simple product. Or more explicitly $R ′ = integral over r ′ in the object space of Tu \left(r′\right) * R \left(r′\right) Tv \left(r′\right)$

Therefore, taking all together, the image values Im(r) computed from vs equals $Im \left(r\right) = D * U \left(r\right) * R ′ * V \left(r\right) * s$

In principle, the vectors U(r) and V(r) are chosen so that, together, they compensate for the effect of Tu and Tv. That is, $Im \left(r\right) = D * R \left(r\right) * s$

Thus, in principle, the computed image values Im(r) of the image corresponds to the reflectivity R(r) at a position "r" in object space can be determined by transmitting e0 and receiving vs. Preferably, U and V compensate at least for the travel time differences of propagation represented by Tu and Tv. Thus the image values approximate R(r). Optionally, U(r) and V(r) also compensate for effects of distance on the signal amplitude.

In principle the correspondence between R(r) and Im(r) applies for an infinite sensing surface with infinitesimally small array elements. In practice both will be finite. As a result, Im(r) will correspond with a wide pass spatially filtered version of R, with some high frequencies suppressed due to the limited size of the sensing surface.

As noted, the vectors U(r) and V(r) are preferably chosen so that, at least together, they compensate for the travel time differences of propagation represented by Tu and Tv. Preferably, U and V at least provide for delay times that vary in the way opposite to the travel times represented by the vectors Tu(r') and Tv(r'). The components Tu(r', p) of the vector Tu(r') correspond propagation from a position r' in object space to the location p of a receiving element. This is a function of the p-r'. The travel time of a ray between be r' and p is | p-r'|/c, with "c" the speed of sound. U(r) may be chosen so that the components U(r, p) of the vector U(r), vary as -|r-p|/c. In the Fourier transform of the time dependence this corresponds to phase factors exp (i f |p-r'|/c ) and exp (-i f | r-p | /c ), with i²=-1 Similar relations hold for V(r).

In an approximation, the dependence of the travel time difference (| r-p|-|p-r'|)/c on p varies as p*(e(r)-e(r'))/c, with p*(e(r)-e(r')) the scalar product of p and the orthogonal projection of the unit vectors e(r) and e(r') in the directions of r and r'. This approximation applies when the ranges of propagation distances |r-p| and |r'-p| are small compared to the object distances |r| and | r'| . In this approximation, the Fourier transforms of the time dependence of the components U(r, p) and V(r,p) of the vectors U(r) and V(r)provides a phase factor exp ( i k*p), where k=2*PI*f e(r)/c, with "f" the temporal frequency. Similarly, in this approximation, the Fourier transform of the time dependence of Tu and Tv contains a phase factor exp ( -i k*p ).

### Sub-sampling

To reduce the time needed for measurement and computation as well as required storage space, sub-sampling may be used. In terms of U and V, sub-sampling can be modeled as a matrix multiplication [U Su], [Sv V] in the vector space with a diagonal matrix Su, Sv with coefficients equal to one or zero. Thus the image values obtained by transmission and receptions can be expressed in terms of the response matrix RT as $Im \left(r\right) = D \left(r\right) * \left[U\left(r\right)Su/\left〈Su\right〉\right] * RT * \left[Sv V\left(r\right)/\left〈Sv\right〉\right] * e$

Herein <Su> is the average value of Su as a function of position, also referred to as the sampling ratio of Su. Similarly, <Su> is the average value of Sv as a function of position.

Sub-sampling results in deviations in the correspondence between the computed image Im(r) and R, i.e. in artifacts. All is well known, one dimensional periodic sub-sampling results in aliasing, i.e. folding back of the signal spectrum, whereby higher frequency components in the non-sub-sampled image give rise to near zero frequency components in the sub-sampled image. The image Im'(r) obtained by sub-sampling can be expressed by error terms compared to the image that is obtainable without sub-sampling: $Im ′ \left(r\right) = Im \left(r\right) + errU + errV + errUV$

With
errU= Integral over r' of D*U(r) (Su/<Su>-I) *Tu(r') R(r') *s
errV= Integral over r' of D*R(r')*Tv(r')*(Sv/<Sv>-I) V(r)*s
errUV= Integral over r' of D* U(r) (Su/<Su>I)*R(r')*Tv(r')(Sv/<Sv>-I)*V(r)*s

In the approximation wherein the dependence of the travel time difference varies as p*(e(r)-e(r'))/c and in the Fourier transform of the time dependence, U(r) (Su/<Su>-I) *Tu corresponds to a spatial Fourier transform of (Su/<Su>-I) and similarly Tv(r')(Sv/<Sv>-I)*V(r) corresponds to a spatial Fourier transform of (Sv/<Sv>-I). Thus, in the case of periodic sampling, effects similar to one dimensional aliasing occur. However, as will be discussed, sampling does not need to be limited to periodic sampling. In principle any selection of locations p may be used as sub-sampling points, even random selection based image points.

### Cross-point images

In principle, U and V may be vectors with as many non-zero response function components as there are array elements 100 in array 10. This would correspond to using all array elements 100 to create transmission beams focused on different positions r in object space and further using all array elements 100 to create selective reception patterns focused on the different positions r.

The actual implementation of the signal flow model may involve successive measurements using successively different (groups of) array elements 100 as transmitters and receivers. This can lead to excessive measurement times.

In embodiment, the images are computed using U and V selected to provide for resolution in the x and y direction respectively, using transmitter elements at different offsets to a row on array 10 and receiver elements in a single row (herein jx and jy will be used to denote the numbers of a selected row and column in matrix 10 and j denotes a combination jx, jy).

In preferred embodiments, the transmitter elements at different offsets to the row lie in a single column. This provides for optimal results. However, mutatis mutandis, the transmitter elements at different offsets may instead lie in a range of columns, selected so that the transmitter elements at different offsets lie no further from a reference column than from the single row. For example, the transmitter elements at different offsets to the row may no more than one or two array elements from the column of the cross-point. In another embodiment, the transmitter elements at different offsets lie along a line transverse to the row direction, e.g. at an angle that deviates no more than thirty degrees from the column direction. The transmitter elements are said to be along such a line if they are the nearest transmitter elements adjacent the line in a row.

In the following, by way of example, preferred embodiments will be described wherein the transmitter elements at different offsets lie in the same column. In this case U and V may be viewed as samples of a single row at jy and a single column at jx respectively, of more general functions U' and V' that depend on two dimensions jx, jy. The access structure of the wearable ultrasound imaging device makes it possible to receive signals in a single row simultaneously.

The image obtained using transmitter elements in a single column of array 10 and receiver elements in a single row may be associated with a "cross-point", i.e. a combination of a row jy and a column jx in array 10. Such an image will be referred to as a "cross-point image" Im(j,r). However, in principle, the image values of such a cross-point image Im(j,r) do not depend on the cross-point "j" and the cross-point image Im(j,r) for any cross-point "j" may be defined over the entire object space, for all positions "r" in that space at any distance from the cross-point "j".

However, when different sub-sampled selections from the transmitter elements in the column jx of array 10 and the receiver elements in the row jy are used for different cross-points, the cross-point images Im(r,j) for different cross-points j will differ from each other, although they approximate the same "true" image. Generally speaking, in plots of slices of Im(j,r) at constant distance to the surface of array 10 the term errU corresponds to artifacts along a horizontal line, the term errV corresponds to artifacts along a vertical line and the term errUV corresponds to artifacts along slanted lines. Because deep sub-sampling may be used, for example sub-sampling of less than one in ten locations on array 10, the deviations can be considerable.

In an embodiment image values of a sum image Im(r) are computed as a sum (preferably a weighted sum, weighted by weight factors w(j)) of image values of such cross point images Im(j, r) $Im \left(r\right) = sum over j of w \left(j\right) Im \left(j, r\right)$

The sum over j is limited to cross point images for only a selection of cross points, for example cross-points on a grid with fixed numbers of columns and rows of array 10 between neighboring cross-points. Different selections of sub-sampling locations on array 10 are used to compute image values for the same position "r" in object space for different cross-point images Im(j,r). As a result, the different deviations due to sub-sampling for different cross-point images will be summed, and the deviations of different cross-point images may counteract each other, or at least will not be the same for each cross-point image. This can be used to reduce the net deviation in the sum image compared to the deviations due to subsampling in the individual cross-point images.

Figure 2c shows part of an array 10 with array elements 100 (only one labeled) only at selected locations that are used to compute the image Im(r) from cross-point images Im(j,r). It should be noted that the array may extend further in all directions from the part that is shown.

Array elements in all rows are shown only for selected columns 120 that contain cross points, and similarly array elements in all columns are shown only for selected rows 110 that contain cross points. Only array elements in the selected rows 110 are used as receivers for the cross-points and only array elements in the selected columns 120 are used as transmitters for the cross-points. No array elements are needed or present at other locations in array 10. In an embodiment, no such array elements are present in the array. However, it should be noted that in other embodiments more array elements may be present, e.g. at all locations in the array, e.g. to enable computation of local images using all array elements in selected areas, when the cross-points are used to compute images of wider areas.

The number of array elements between successive cross-points is merely an example for the sake of illustration. In practice cross-points 10 to 100 array elements may be present between successive cross-points. All of the cross-point images may be computed according to the same method, a distinction being made only in the selections of sampled locations in array 10 that are used to compute the different cross-point images. In an embodiment, the general vectors U(r) and V(r) used in this computation are the same for all of the cross-point images, but different selections of components of these vectors are used for respective different cross-points, according to selections of sampled locations in array 10 for these respective cross-points. In a preferred embodiment, this selection is made in the first place so that the image values of each cross point image Im(j, r) are obtained by transmitting ultrasound only from locations of array elements 100 in the column jx and using only array elements in the row jy. In the second place, for each cross point image Im(j, r) selections are made of locations in this row and column that are used to compute the cross-point image.

Each cross-point image is based on transmission from array elements at sub-sampled locations in a range in a column of array 10 and reception from array elements at sub-sampled locations in a range in a row of array. The subs-sampled locations include locations at the ends of the ranges. The ranges in the row and the column for two of the cross-points 130, 132 are shown as two dimensional cross-hair windows with a horizontal range 140a, 142a and a vertical range 140b, 142b on array 10.The ranges for these cross points are so large that they overlap each other. As shown, each range in the cross-hair windows 140a, b, 142a, b for a cross-point 130, 132 includes that cross-point. Preferably, the crossing of the ranges 140a, b, 142a, b of each cross point 130, 132 at least lie close to the cross-point 130, 132.

By way of example, the array may have size of 150x150 mm, with 5-20 array cells per mm, and the ranges may be 20-100 mm long.
Using cross-point images obtained with transmission and reception with array elements dispersed over a much wider range of locations than the distance between cross-point has the effect of providing high resolution in the cross-point images.

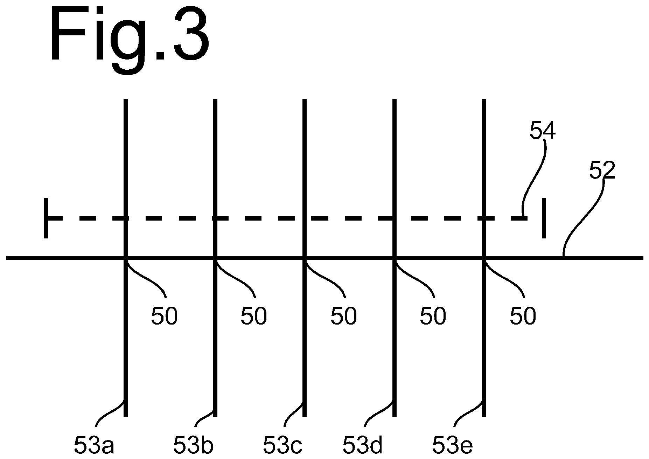

Figure 3 similarly illustrates cross-point locations 50 along a single horizontal line 52. Different cross-point locations are associated with a respective vertical line 53a-e through the cross-point location. The image for each cross-point location 50 is formed using excitation signals from array elements 100 in a first a-periodically subsampled selection of array element locations along its vertical line 53a-e and reception signals from array elements 100 in a second a-periodically subsampled selection of locations along the horizontal line 52.

For the cross point location 50 of one of the vertical lines, figure 3 shows the horizontal range 54 along horizontal line 52 between the leftmost and rightmost location in the second selection for that cross-point location 50. This horizontal range 54 includes the cross-point location 50 and extends beyond the neighboring cross-point locations 50 in the row direction. Thus, at least for nearest neighboring cross-points in the row direction, the horizontal ranges of locations at which signals are received for the cross-points overlap. The locations in the first sampled selection similarly extend over a similar vertical range that includes the cross-point location 50 and preferably at least the vertical ranges of the nearest neighboring cross-points in the column direction.

For each cross-point location 50 labeled "j" ultrasound signals time dependent signals e0(i,j) are excited at selected locations labeled jy along the vertical line 53a-e of the cross point location j=(jx,jy). Similarly for each cross-point location j ultrasound signals time dependent signals vs(jx, jy) are received and a selection locations labeled jx along the horizontal line 52 of the cross point location j is used to compute the image values Im(r,j) of the image for the cross-point. The image values for the same "r" of cross-point images for different cross-points are summed to form the image value of a sum image.

In the sum image, the deviation due to sub-sampling is the sum of the deviations errU, errV, errUV in the cross-point images. errU and errV show up as deviations along the x-axis and the y axis in the image respectively. errUV shows up as deviations on slanted lines in x-y planes in the image. The expression for errU and errV of the sum image, is the same as for the cross-point images, except that the sums of the sampling functions take the place of the sampling functions Su(j)/<Su(j)>, Sv(j)/<Sv(j)> for the cross-points: e.g. 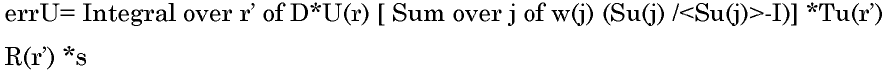 errU= Integral over r' of D*U(r) [ Sum over j of w(j) (Su(j) /<Su(j)>-I)] *Tu(r') R(r') *s

The size of the deviation decreases when the sum over j of w(j) Su(j)/<Su(j)> better approximates a constant function, i.e. when the sampling function equals one for more locations on array 10. The deviations are large for an individual cross-point image when deep sub-sampling is used, because in that case the value of w(j) Su(j)/<Su(j)> is far from a constant function.

But as the expression for errU shows, the terms w(j) Su(j)/<Su(j)> from different cross-point images add up, so that the use of different subsampling selections for different cross-points can be used to improve the approximation of a constant function. The more the sub-sampling locations are interspersed, the less the selections of sampling locations for the different cross-point image "j" coincide, the smaller the deviation due to subsampling in the sum image.

The variance of the sum over j of (Su(j)/ <Su(j)>-1) may be used as a measure for the approximation of a constant function. For a single cross point image, wherein Su(j) equals one for a sub-sampled selection of m of the n possible sampling locations, the variance of (Su(j)/<Su(j)-1> per location is 1/<Su(j)>-1. For N overlapping cross-point images without coinciding sampling points the variance is (1/N*<Su>-1 (the absence of coinciding sampling points implies that N*<Su> is less than or equal to one), i.e. the variance decreases with the number N of cross-points with overlapping ranges. It should be noted that neighboring cross-points in both the x and y directions count for N in this context, so that rapidly increasing gains can be realized with a two-dimensional array of cross-points.

Mutatis mutandis the same applies for the deviation errV. For errUV the expression is more complicated, but it has been found that reducing the coincidences of Su and Sv in different cross-point images also reduces errUV for the sum image. For both, the gain is optimal when the direction of along which the tranmsittion elements are located is perpendicular to the row direction.

Accordingly, to make it possible that the net deviation in the sum image is smaller than deviations in the individual cross-point images due to sub-sampling, the ranges on array 10 that are associated with neighboring cross-points 130, 132 overlap and the sub-sample locations for the different cross-point in the overlap lie at least partly interspersed with each other. Locations of a first set of locations are said to be interspersed with locations of a second set of locations when different locations of the first set lie between different pairs of successive locations of the second set, not excluding that some pairs of successive locations of the second set have pluralities of locations of the first set lie between them, nor excluding that some pairs have no locations of the first set lying between them. When the locations of the first and second set are dispersed over a same range of locations, they generally interspersed.

The ranges associated with a cross-point 130 may have overlap with a plurality of the ranges associated with nearby cross-points locations, e.g. with windows associated with N nearby cross-points in both the x and y direction, with N=2 or N=4 for example. Preferably, the ranges for pairs of nearest neighbor cross-points are at least twice as large as the distance between the cross-points. More preferably three, four or more times as large reduced resolution. A sliding window may be used, with the same size for each cross point, and the same relative location to the cross-point, although the sampling locations within the window relative to the cross-point may differ.

The number of sub-sample locations in each range may be one in five of the total number of sub-sample locations in the range for example, or less, e.g. one in twenty. Preferably, the sub-sampling rate is so large that on average at least two sub-sample locations for a cross-point lie between that cross-point and each of its nearest neighboring cross points. Preferably, at least thirty two sub-sample locations are used in each range. Larger numbers such as a hundred twenty eight or two hundred fifty six may be used in each range.

When the sub-sampling locations are dispersed over the entire sampling range, the effect of sub-sampling on resolution is limited. The widths of the zero-frequency peak of the absolute square of the Fourier transform of the sampling functions Su, Sv can be used as a measure both of resolution and of dispersment of the sub-sampling locations. Preferably, Su, Sv are selected so that this width is no more than twice the width without sub-sampling.

In an embodiment, the selection of the sampling locations in the horizontal and vertical ranges 140a, b of the window for a cross-point 130 is made so that none of the sampling locations in the horizontal and vertical ranges 140a, b of the window 140 coincides with any sampling location for other cross-points 132 with the horizontal and vertical ranges 142a, b windows (e.g. 142) that overlap with those for the cross-point 130. With low sub-sample rates this can easily be realized. The sampling locations in each range lie at locations that are interspersed with sampling locations in overlapping ranges for other cross-points. This reduces the net deviation in the sum image. Deep sampling, e.g. using less than one in ten of the locations in the sampling range, makes it possible to make such a selection even if there is a large number of overlapping windows.

However, the net deviation in the sum image can be smaller than the deviation in a cross-point image even if some of the sampling locations in the window 140 coincide with those in windows with overlapping ranges 142a,b, as long as not all sampling locations in the overlap coincide. That is, at least a part of the sampling locations in a window lie interspersed with sampling locations in overlapping windows, but another part of the sampling locations in the window may coincide with sampling locations in overlapping windows. Preferably at least half the sampling locations in a window 140 do not coincide with sampling locations in overlapping windows. Similarly, it is not necessary that all possible sampling locations are used in the ranges 140a,b, 142a,b of at least one window .

### A-periodic sub-sampling

Preferably, the selections are a-periodic, i.e. the selected sampling locations for a cross-point do not lie at integer multiples of a base distance. A-periodic selection has the advantage that in the remaining net deviation in the sum image aliasing effects are smeared out, reducing the size of the deviation at the image locations, r, where the size of the deviation is maximal.

The Fourier transform of the location dependence of Su-<Su> and/or Sv-<Sv> can be used to characterize a-periodicity. The subtraction of Su, Sv prevents a peak at zero frequency corresponding to deviation free sampling. In the approximation wherein the dependence of the travel time difference varies as p*(e(r)-e(r'))/c, the successive operations U(r) Su Tu result in deviations that correspond to a convolution of R with this Fourier transform. In the case of periodic sub-sampling, the amplitude of this Fourier transform has a sharp peak at the sampling function, which gives rise to aliasing. In contrast, in the case of aperiodic sub-sampling the amplitude is distributed quasi continuously over a range of frequencies. One measure Q of a-periodicity due to reception sampling Su is the value of the highest peak, except for the peak at zero frequency, if the absolute square of the Fourier transform of the sampling function Su, divided by the maximum value of the absolute square of the Fourier transform of the periodic subsampling function with the same number of sub-sample locations (the same sub-sampling rate). Preferably an a-periodic sub-sampling with a Q smaller than 0.5 is used and more preferably with a Q less than than 0.1. Mutatis mutandis, a similar criterion may be used for Sv. Sampling selections that satisfy such criteria are easy to achieve with deep sub-sampling: when <Su> is smaller than 0.5, almost any random selection of sample locations will suffice.

Thus, using sub-samples dispersed over a much wider range than the distance between cross-point serves to provide high resolution in the cross-point image, the measurement time needed for using such wider ranges is reduced by sub-sampling and the resulting artefacts are reduced by a-periodic sub-sampling and summing cross-point images obtained with substantially different sub-sampling selections.

In one embodiment, the a-periodic selection may be obtained by using frequency modulation, wherein successive sampling locations p(m) indexed by m are chosen according to p(m+1)=p(m) + r(m). Herein the frequency r(m), i.e. the distance between successive sampling points, is selected so that the selected distances are distributed over a predetermined range of distances (the frequency modulation range), preferably excluding distances below a predetermined distance, so that successive sampling locations within that predetermined distance are prevented. The frequencies r(m) may be randomly selected according to a predetermined probability distribution over the frequency modulation range. Thus, on average <Su> is the average of the probability distribution. By way of example, the probability distribution may be constant in a limited range centered on <Su> and zero elsewhere).

Even when the sampling selection for each cross-point is strictly periodic (p(m+1)=p(m) + d, with "d" the sampling period), so that the Q value equals one, the net deviation in the sum image can be reduced by selecting the sampling locations for computing different cross-point images differently, e.g. by using different values of p(m) mod d for different cross point images. However, using a-periodic subs-sampling has been found to provide for better suppression of the deviations.

Although an embodiment has been described wherein the cross point images are each computed with reception and transmission along a single row and column respectively, it should be noted that, instead of a single row a third selection of a plurality of rows may be used instead. This can be viewed to involve a plurality of cross-point for the same column and different rows, except that the same sampling locations are used in the column. Different selections may be used for the different rows in the third selection, preferably so that at least part of the sampling locations are interspersed with the sampling locations in the different second selections, as well as with the sampling locations for other cross-point images. Alternatively, this can be viewed as a single cross-point image involving further rows in addition to the row that defines its cross-point.

Using the same sampling locations in the column for the plurality of rows instead of using different selections in the column for each row may increase the deviations in the sum image, but is still better than using a single cross-point image.

### Cross-points

In the illustrated embodiments, a regular grid of cross-points may be used, e.g. with successive cross-points at a distance of 10-100 array elements along the rows and columns. When a regular grid of cross-points is used, the selected rows 110 and selected columns 120 form a regular grid of horizontal and vertical lines. Although figure 2c shows an example wherein one in four rows and column are selected rows 110 selected columns 120 wherein cross-points are located respectively, other (generally larger) distances between selected rows 110 and selected columns 120 may be used. However, a regular grid is not necessary. An irregular grid may be used instead. This may further reduce deviations due to sub-sampling

It should be noted that the cross-point images are not merely separate image patches that at most partly overlap with the cross-point image patches of neighboring cross-points that are not merely stitched together. In principle, the cross point images each define image values in the same image space and summing cross-point images obtained with substantially different sub-sampling selections is used to reduce subsampling artefacts in high resolution images obtained with reduced measurement time.

The weights may be cross-point independent. Alternatively, the weight factors w(j) may be used to give variable weights to different cross-point images, e.g. dependent on the position "r" in the object space for which the sum image is computed. In other embodiments, the weights w(j) may be used to give less or no weight to different cross-points with increasing distance from an image point, e.g. as a decreasing function, or at least nonincreasing, function of the distance between the image position r and the cross-point location. Alternatively, the image values of cross-point images for cross-points "p" at large distances from the image point "r" may simply be ignored when computing the sum image for that image point. For example, the weights may be made non zero (e.g. constant) only for a cross-point that is closest to the image position for which the image value is computed and for all other cross-points with sub-sample ranges that overlap with the range for that cross-point. This can be used to limit noise when reducing sub-sampling artifacts.

The weights may be chosen so that their sum over the cross-points equals one, in order to normalize the sum image. However, normalization is not necessary, since a non-normalized image is also an image. A useful image can be obtained even if the weights w(j) are omitted.

For a large array, wherein the length of the rows and/or columns is not much smaller, or even larger, than the distance to object positions "r", use of a weighted sum of cross-point images to compute the sum image may serve to give decreasing weights to cross-point images with increasing distance between the cross-point and "r". The use of a sum of cross point images that, in principle, represent the same object space makes it easy to account for the fact that the most reliable cross-point images are obtained for cross-points at the shortest distances to "r".

### Device array implementation

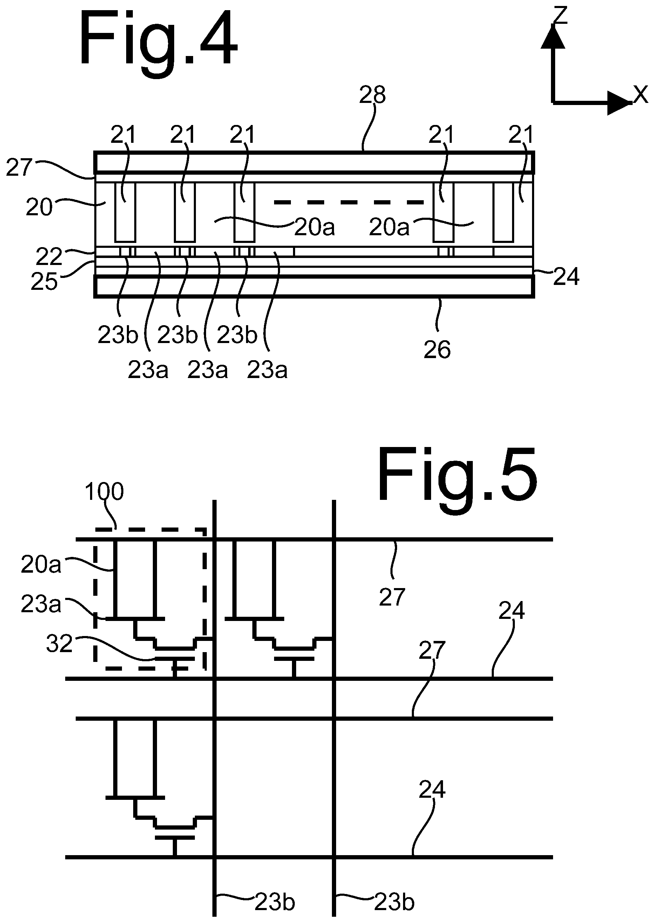

Figure 4 shows a cross-section in a side view of the device. In the exemplary example, each array element 100 comprises a part of a layer 20 of piezo electric material, wherein the part of layer 20 in array element forms a piezo electric pillar 20a that is separated laterally from the parts of layer 20 in other array elements by spaces 21 which may contain air or a nonpiezo electric filling material. By way of example, each piezo electric pillar 20a may be eighty micrometer high and have a square cross-section with forty micrometer long sides. Successive array elements 100 may be provided at a pitch of sixty micrometer.

Spaces 21 may be created for example by starting from a continuous layer by hot embossing using a lithographically pattern stamp. An electric conductor layer 27 is provided on top of the piezo electric pillars 20a, extending over array 10. Furthermore, each array element 100 has its own electrode 23a formed in an electrode layer 22 below the piezo electric pillar 20a, for applying an electric field through its piezo electric pillars 20a between an electrode 23a and conductor layer 27.

The substrate on which array 10 is located may be a flexible carrier foil 26. Optionally an encapsulation layer 28 is provided over electric conductor layer 27. Row selection circuit 12 and column access circuits 14, 16 are located adjacent array 10 on flexible carrier foil 26.

Row lines 24 may be provided below electrode layer 22 coupled to row selection circuit 12. Column lines 23b may be provided in electrode layer 22, coupled to column access circuits 14, 16.

Each array element 100 may comprise an access transistor with a channel formed in a transistor layer 25, the channel being coupled between the electrode 23a of the array element 100 and the column lines 23b for the column of array 10 in which the array element 100 is located. The row line 24 for the row of array 10 in which the array element 100 is located may be connected to the gate of the access transistor of the array element 100, or form the gate itself.

Figure 5 shows an exemplary circuit diagram of an array element 100 (indicated by a dashed rectangle) coupled to a row line 24 and a column line 23b. Array element 100 comprises a piezo electric pillar 20a, an electrode 23a and an access transistor 32. Piezo electric pillar 20a is located between electrode 23a and electric conductor layer 27, of which the latter may be at ground potential. The channel of access transistor 32 is connected between electrode 23a and the column line 23b of the column of array 10 in which the array element 100 is located. The gate of the transistor is connected to the row 24 of array 10 in which the array element 100 is located.

In some embodiments, all array elements 100 may be designed and wired to act both as ultrasound transmitters and as ultrasound receivers. Alternatively, different array elements 100 may be designed and wired to implement only one of transmission and reception.

It may be noted that, at an abstract level, the circuit of the ultrasound imaging device 42 has some similarity to a DRAM memory circuit or a pixel based display control circuit. But its use differs at least in that time dependent signals are used to generate and measure ultrasound signals.

### Processing

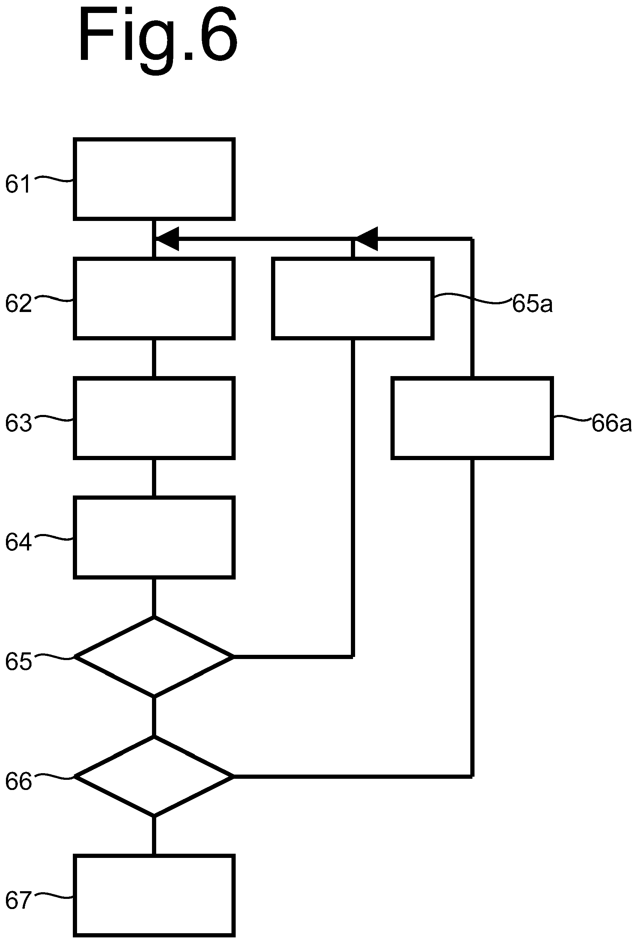

Figure 6 shows a flow chart of an embodiment of operation of the imaging system. In a first step 61 processing system 40 selects a cross point location (e.g. 130 figure 2c) indexed by j=(jx,jy). In a second step 62 processing system 40 supplies an address to cause a first column access circuit to allow a selection signal from signal generator 44 to pass to the column line 23b corresponding to the cross point location 130.

In a third step 63, processing system 40 causes the row selection circuit to apply a selection signal to the row line 24 to select the row corresponding to a location in the first selection of sub-sampled locations from the vertical range 140 for the cross-point location 130. While the selection signals are applied, processing system 40 causes signal generator 44 to generate an ultrasound excitation signal according to the component of e0(t) that corresponds to the selected array element, which the first column access circuit passes to the column line 23b. Under control of the row line signals this excitation signal is passed selectively to the piezo electric pillar of the array element 10 at the selected row and column. For example, the excitation signal may be a pulse modulated signal; the delay corresponding to the selected component of e0(t) may be accounted for separately.

Subsequently, in a fourth step 64, processing system 40 causes the row selection circuit to apply signals to the row controls to the row lines 24 to select a row corresponding to the row in which the horizontal range 140 of the selected cross-point 130 is located. Furthermore, processing system 40 causes a second column access circuit to allow a selected one or a selected plurality of received time dependent signals from the array elements in the columns that correspond to the locations in the second selection of subsampled locations from the horizontal range 140 for the cross-point location 130 to pass to detector circuit 45. In response detector circuit 45 obtains digitized signal values for a sequence of time points from transmission of the ultrasound excitation signal. Preferably, signals from a plurality of columns are passed to and handled by detector circuit 45 simultaneously. In an embodiment, the second column access circuit is configured to pass at least the signals from the horizontal range of a selected cross-point simultaneously. In another embodiment, the second column access circuit may be configured to pass the signals from programmed selected columns corresponding to the sub-sampled locations in the row simultaneously. Still in fourth step 64, processing system 40 reads the digitized signals from detector circuit 45 and stores the signals in a memory (not shown).

Fourth step 64 may be executed starting immediately after the ultrasound excitation signal of third step 63 ends. Fourth step 64 may continue during a time interval in which measurable reflections of the ultrasound excitation signal are received. If received signals from only a single column can be processed by detector circuit 45 at a time, third to fourth step 63, 64 may be repeated for successive locations in the second selection of sub-sampled locations from the horizontal range 140 for the cross-point location 130.

In a fifth step 65, processing system 40 tests whether all locations in the first selection of sub-sampled locations from the vertical range 140 for the cross-point location 130 have been used. If not, processing system 40, in a step 65a, selects a next location from the first selection and repeats from second step 62 for the next location. When all locations in the first selection 52 have been used, processing system 40 performs a sixth step 66, testing whether the previous steps have been executed for all cross point locations 130. If not, processing system 40 selects a next cross point location 132 in step 66a and repeats from second step 62 for the next cross point location. Once it is determined in sixth step 66 that the preceding steps have been executed for all selected cross point locations, processing system 40 executes a seventh step 67 wherein processing system 40 computes an image.

The image computation of seventh step effectively involves applying U(j, r) and V(j, r) for each image position "r" and applying D to the results to obtain cross-point image values for each cross-point "j" , followed by (weighted) summing of the cross-point image values obtained for the same image position "r" in the cross-point images for different cross-points j. The effect of V(j, r), which formally represents a vector with components for different array elements that represent signals transmitted from these array elements, can be synthesized by summing signals received in response to transmissions by individual array elements. Similarly, the effect of U(j, r) can be synthesized by summing signals received by individual array elements. Because V(j, r) does not vary with the x-component of "r" and U(j, r) does not vary with the y-component of "r", part of the computation of the cross-point image values for different positions "r" can be shared, to reduce the amount of computation of the of the cross-point image values.

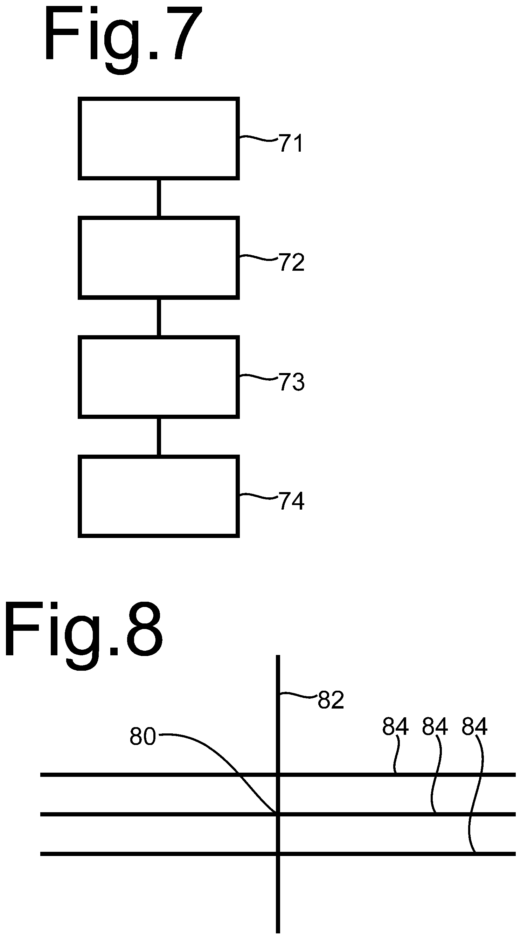

Figure 7 illustrates an example of an image computation that may be used in seventh step 67. In this example, position dependent cross-point images are computed first, and summed afterwards. In a first step 71, for each cross point location j, synthesized signals resulting from a combination of transmissions from the first selection of locations of sub-sampled locations from the vertical range 140 for the cross-point location j are synthesized for respective locations in the second selection of sub-sampled locations from the horizontal range 140 for the cross-point location j.

As will be remembered, in signal processing flow terms, the excitation signal for measuring reflection at a position r in object space is defined in terms of a response function vector V(j, r), with vector components that correspond to locations in the array and define time or frequency domain filtering operations. Only selected components of V, corresponding to a first subsampling selection of locations in the array are used, with different first subsampling selections for different cross-points. Similarly, only a selection of sub-sampling selection of locations in the array are used.

Accordingly, processing system 40 synthesizes the synthesized reflection for each location in the second selection by summing results of applying the components of the first direction response function vector V(j, r) to all selected measured reflection signals obtained for that location in the second selection in response to transmissions from different locations from the first selection of locations 50 for the cross point location.

The measured reflection signals and the synthesized reflection signals depend on time, and the synthesized reflection signals may be computed for a plurality of time points. Alternatively a Fourier transform domain computation may be used to account for the time dependence, including phase factors that treat the Fourier transforms domain signals as complex numbers so as to compensate travel time differences in the beam direction from different locations 50 from the first selection.

In a second step 72, for each cross point location processing system 40 synthesizes reflection signals for object space directions from the synthesized reflection signals for the locations from the second selection for the cross point location.

As will be remembered, in signal processing flow terms, the received signal for measuring reflection at a position r in object space is defined in terms of a response function vector U(j, r), with vector components that correspond to locations in the array and define time or frequency domain filtering operations. Only selected components of U, corresponding to a second subsampling selection of locations in the array are used, with different second subsampling selections for different cross-points.

Accordingly, processing system 40 synthesizes the reflection signals for object space directions by summing results of applying components of the second direction response function vector U(j, r) to all synthesized reflection signals obtained for locations in the second selection for the cross point location. The synthesized reflection signals depend on time, and may be computed in the Fourier transform domain.

Since the first selection selects a plurality of locations in a same column, the first step 71 provides for synthesized increased direction sensitivity when the component of the direction along the column direction varies. Similarly, since the second selection selects a plurality of locations in a same row, the second step 72 provides for synthesized increased direction sensitivity when the component of the direction along the row direction varies. First and second step 71 compensate for direction dependent propagation time difference, but the synthesized signals may still depend on time from transmission of the ultrasound excitation signal, e.g. due to variation of reflectivity in the object space as a function of distance to the array.

In a third step 73, for each cross point location, processing system 40 uses the synthesized signals for different combinations of beam and reception directions to obtain reflection measurements for a selected time delay from the excitation of the ultrasound signal. This corresponds to the effect of the signal processing operation "D".

In principle, image values of a three dimensional image of ultrasound reflection in the object space can be computed in this way. Optionally, the computation may be limited to positions on a surface in object space. In a fourth step 74, processing system 40 uses the results for different cross point locations to compose a sum image of the reflections. Optionally the results may be obtained along a conformal surface(s) that is (are) conformal to the surface of the array at a distance(s) d from the surface of the array, using the results of the third step 73 for locations in the conformal surface(s). Another option is to determine results for a two-dimensional slice of object space that extends transverse to the array.

As noted, the example of figure 7 serves primarily for the sake of illustration of an implementation of the computations. In practice different implementations could be used. For example, part or all of time and/or spatial position dependent computations may be replaced by computations in a Fourier transform domain. As another example, the sequence of the steps could be changed, e.g. synthesizing signals for reception directions first and for beam directions later, or by selecting signals for the time delays earlier and synthesizing signals for reception directions and/or beam directions later. As another example, first and second steps 71, 72 could be performed as part of steps shown in figure 6, as the necessary signals have been recorded for a cross point location, but before all signals have been recorded. For example, if second step 72 is performed first, it could be performed before signals using all locations 50 from the first selection have been used, or steps 71, 72 could be performed before all cross point locations have been used.

In an embodiment, the total measurement time may be reduced by receiving responses to the same transmitted signal at a plurality of rows. The transmission time interval during which an array element is excited to transmit ultrasound is usually much shorter than the reception time interval during which reflections of that ultrasound are received. In most embodiments, it is possible to change the row that is selected many times during the reception time interval, to provide for time-multiplexed output of reflections at different rows.

Thus, as illustrated in figure 8, signals for different cross-points along a same column 82 may be received with receivers in different rows 84 in response to the same transmitted signal. Receiving signals for different cross-points along a same column 82 with receivers in different rows 84 has the drawback that the same vertical sub-sampling selection will be used for the different cross-points, which makes the suppression of sub-sampling artifacts less effective. But in many applications some reduction suppression of sub-sampling artifacts may be tolerated.

Alternatively, this may be viewed as an embodiment wherein receivers in multiple rows 84 are used for the same cross-point 80, to obtain part of the resolution in the column direction, thereby reducing the number of locations in the column direction that would be needed on their own to provide for the same resolution. Thus, a plurality of rows 84 of receivers is used for a cross-point, so that one or more of these rows 84 do not cross the column 82 at the cross point 80.

In this embodiment, the locations of the array elements that are used for reception in different rows 84 are used together with the locations of array elements that are used for transmission in different rows of the column 82 to synthesize the resolution in the column direction. In this embodiment, the resolution in the column direction can be described with the product of a transmission function and a reception function, which are a result of synthesis using array elements for transmission and using array elements for reception respectively.

As in the embodiment with a single row, the response functions U(j, r) may be selected to compensate for travel time differences from the locations of the array elements that are used for reception (now also in different row) to the position "r" in object space for which the image is formed.

## Claims

1. A method of forming an object image of ultrasound reflectivity in an object space, using an array (10) of rows (110) and columns (120) of ultrasound transducers (100) that define a sensing surface adjacent the object space, the method comprising,
- computing object image values for each position (r) in the image of ultrasound reflectivity (R, R(r)) in the object space by summing cross-point based image values (Im(j, r)) for the position (r) determined for a plurality of cross-points locations (j, 50, 80, 130, 132) that are each associated with a respective combination (j) of a row and column in the array (10);
wherein the cross-point based image value (Im(j, r)) for each respective cross point location (j, 50, 80, 130, 132) of the plurality of cross point locations is determined by
• transmitting ultrasound transmission signals (e0) from the ultrasound transducers (100) at a subsampled first selection of first sampling locations that lie no further from the column (120) of the respective cross point location (130) than from the row (110) of the respective cross point location (130), the first selection extending over a first range (140b, 142b) of offsets from the row of the first sampling locations;
• receiving reflections (vs) of the ultrasound transmission signals (e0) from the object space at a subsampled second selection of second sampling locations along the row (24, 110, 52) of the respective cross point location, or second selections of second sampling locations in a subsampled third selection of rows (84) that includes the row of the respective cross point location (130), the second selection or second selections extending over a second range (54, 140a, 142a) of the second locations along the row of the respective cross point location, at least part of reflections (vs) in the second selection or selections being received simultaneously;
• computing, for the respective cross point location (j, 50, 80, 130, 132), the cross-point based image value (Im(j, r)) associated with reflectivity (R, R(r)) at the position (r) in the object space, synthesized from the reflections (vs) received at the second selection or second selections in response to the ultrasound transmission signals (e0) at the first selection;
wherein
- the first range (140b) for each respective cross point location (130) has a first overlap with the first ranges (142b) of more than one of the plurality of the cross point locations (132), at least part of the first locations of the first ranges (142b) of the more than one of the plurality of the cross point locations (132) lying at least partly interspersed between the first locations in the first range (140b) for the respective cross point location (130), and
- the second range (140a) or the second ranges for the respective cross point location (130) has a second overlap with the second ranges (142a) of more than one of the plurality of the cross point locations (132), at least part of the second locations of the second range (142a) of the more than one of the plurality of the cross point locations (132) lying at least partly interspersed between the second locations in the second range (140a) for the respective cross point location (130).

2. A method according claim 1, wherein the first range (140b, 142b) for the respective cross point location (130) extends at least to the offset of the nearest cross-point location (132) from the respective cross point location (130) and/or the second range (54, 140a, 142a) for the respective cross point location (130) extends at least to the nearest row of the respective cross point location (130).

3. A method according to any of the preceding claims, wherein the first selection and the second selection are a-periodic selections.

4. A method according to claim 3, wherein values of distances (r(m)) between successive ones of the first sampling locations and/or between successive ones of the second sampling locations lie distributed over a predetermined distance value range that contains a plurality of distance values.

5. A method according to claim 4, wherein a lower limit of the predetermined distance value range is at most half an average of the values of the distances.

6. A method according to any of the preceding claims, wherein the cross points (j, 50, 80, 130, 132) are located on a two-dimensional periodic grid of locations, with a period smaller than the first (140b, 142b) and second (54, 140a, 142a) ranges.

7. A method according to any of the preceding claims, wherein average sub-sample ratios of the first and second selections are at most half a density of the cross points (j, 50, 80, 130, 132) along the column (120) and/or row (110) of the respective cross point location.

8. A method according to any of the preceding claims, wherein said summing of the cross-point based image values (Im(j, r)) for the position (r) is a weighted summing (w(j)).

9. A method according to any of the preceding claims, wherein the weighted summing (w(j)) defines a window of cross-point locations (j, 50, 80, 130, 132) of which the first (140b, 142b) and/or second (54, 140a, 142a) ranges all overlap with the first (140b, 142b) and/or second (54, 140a, 142a) range of at least one of the cross-point locations in the window.

10. A computer program product, comprising a program of instructions for a programmable processing system (40), which, when executed by the processing system (40) of the ultrasound imaging system according to any of claims 11 to 15, cause the processing system (40) to perform the method of any one of the preceding claims.

11. An ultrasound imaging system, comprising
- an array (10) of rows (110) and columns (120) of ultrasound transducers (100) that define a sensing surface;
- an excitation circuit (12, 16, 44) configured to cause ultrasound emission from selectable first ultrasound transducers (100) in the array (10);
- a reception circuit (12, 14, 45) configured to receive ultrasound measurement signals (vs) from selectable second ultrasound transducers (100) in the array (10);
- a processing system (40) configured to
- compute object image values for each position (r) in an image of ultrasound reflectivity (R, R(r)) in the object space by summing cross-point based image values (Im(j, r)) for the position (r) determined for a plurality of cross-points locations (j, 50, 80, 130, 132) that are each associated with a respective combination (j) of a row and column in the array (10);
wherein processing system (40) is configured to determine the cross-point based image value (Im(j, r)) for each respective cross point location (j, 50, 80, 130, 132) of the plurality of cross point locations by
• causing the ultrasound transducers (100) to transmit ultrasound transmission signals (e0) from at a subsampled first selection of first sampling locations that lie no further from the column (120) of the respective cross point location than from the row (110) of the respective cross point location, the first selection extending over a first range (140b, 142b) of offsets from the row of the respective cross point location;
• receiving reflections (vs) of the ultrasound transmission signals (e0) from the object space at a subsampled second selection of second sampling locations along the row (24, 110, 52) of the respective cross point location, or second selections of second sampling locations in a subsampled third selection of rows (84) that includes the row of the respective cross point location, the second selection or second selections extending over a second range (54, 140a, 142a) of the second locations along the row of the respective cross point location, at least part of reflections (vs) in the second selection or selections being received simultaneously;
• computing, for the respective cross point location (j, 50, 80, 130, 132), the cross-point based image value (Im(j, r)) associated with reflectivity (R, R(r)) at the position (r) in the object space, synthesized from the reflections (vs) received at the second selection or second selections in response to the ultrasound transmission signals (e0) at the first selection;
wherein
- the first range (140b) for each respective cross point location (130) has a first overlap with the first ranges (142b) of more than one of the plurality of the cross point locations (132), at least part of the first locations of the first ranges (142b) of the more than one of the plurality of the cross point locations (132) lying at least partly interspersed between the first locations in the first range (140b) for the respective cross point location (130), and
- the second range (140a) or the second ranges for the respective cross point location (130) has a second overlap with the second ranges (142a) of more than one of the plurality of the cross point locations (132), at least part of the second locations of the second range (142a) of the more than one of the plurality of the cross point locations (132) lying at least partly interspersed between the second locations in the second range (140a) for the respective cross point location (130).

12. An ultrasound imaging system (42) according to claim 11, wherein
- the excitation circuit (12, 16, 44) comprises a signal generator (44) and a first access circuit (16) coupled between the signal generator (44) and columns (120) of the array (10), to generate ultrasound with a time dependence defined by the signal generator (44) from the first selectable locations in the array (10), the first access circuit (16) being configured to select locations of the first ultrasound transducers (100) under control of the processing circuit (40);
- the reception circuit (12, 14, 45) comprises a detection circuit (45) and a second access circuit (14) coupled between columns (120) of the array (10) and the detection circuit (45), the detection circuit (45) being configured to detect a time dependence of the ultrasound measurements (vs) from the selectable second ultrasound transducers (100) in the array (10), the second access circuit (14) being configured to select locations of the second ultrasound transducers (100) under control of the processing circuit (40).

13. An ultrasound imaging system (42) according to claim 11 or 12, wherein the first (140b, 142b) and/or second (54, 140a, 142a) ranges for the respective cross point location (130) extend at least to the nearest cross-point locations (132) from the respective cross point location (130) along the column (120) and/or row (110) of the respective cross point location respectively.

14. An ultrasound imaging system (42) according to any of claims 11-13, wherein the first selection and the second selection are a-periodic selections.

15. An ultrasound imaging system (42) according to any of claims 11-14, wherein said summing of the cross-point based image values (Im(j, r)) for the position (r) is a weighted summing (w(j)).

## Patentansprüche

1. Verfahren zum Erzeugen eines Objektbildes einer Ultraschallreflektivität in einem Objektraum unter Verwendung eines Arrays (10) aus Zeilen (110) und Spalten (120) von Ultraschallwandlern (100), die eine an den Objektraum angrenzende Erfassungsfläche definieren, wobei das Verfahren umfasst:
- Berechnen von Objektbildwerten für jede Position (r) in dem Bild der Ultraschallreflektivität (R, R(r)) in dem Objektraum durch Summieren kreuzungspunktbasierter Bildwerte (Im(j, r)) für die Position (r), die für eine Mehrzahl von Kreuzungspunktpositionen (j, 50, 80, 130, 132) bestimmt werden, die jeweils einer jeweiligen Kombination (j) aus einer Zeile und einer Spalte in dem Array (10) zugeordnet sind;
wobei der kreuzungspunktbasierte Bildwert (Im(j, r)) für jede jeweilige Kreuzungspunktposition (j, 50, 80, 130, 132) der Mehrzahl von Kreuzungspunktpositionen bestimmt wird durch
• Senden von Ultraschallübertragungssignalen (e0) von den Ultraschallwandlern (100) an einer unterabgetasteten ersten Auswahl erster Abtastpositionen, die nicht weiter von der Spalte (120) der jeweiligen Kreuzungspunktposition (130) entfernt liegen als von der Zeile (110) der jeweiligen Kreuzungspunktposition (130), wobei sich die erste Auswahl über einen ersten Bereich (140b, 142b) von Versätzen von der Zeile der ersten Abtastpositionen erstreckt;
• Empfangen von Reflexionen (vs) der Ultraschallübertragungssignale (e0) aus dem Objektraum an einer unterabgetasteten zweiten Auswahl zweiter Abtastpositionen entlang der Zeile (24, 110, 52) der jeweiligen Kreuzungspunktposition oder an zweiten Auswahlen zweiter Abtastpositionen in einer unterabgetasteten dritten Auswahl von Zeilen (84), die die Zeile der jeweiligen Kreuzungspunktposition (130) einschließt, wobei sich die zweite Auswahl oder die zweiten Auswahlen über einen zweiten Bereich (54, 140a, 142a) der zweiten Positionen entlang der Zeile der jeweiligen Kreuzungspunktposition erstrecken und zumindest ein Teil der Reflexionen (vs) in der zweiten Auswahl oder den zweiten Auswahlen gleichzeitig empfangen wird;
• Berechnen, für die jeweilige Kreuzungspunktposition (j, 50, 80, 130, 132), des kreuzungspunktbasierten Bildwertes (Im(j, r)), der der Reflektivität (R, R(r)) an der Position (r) in dem Objektraum zugeordnet ist und aus den an der zweiten Auswahl oder den zweiten Auswahlen als Reaktion auf die Ultraschallübertragungssignale (e0) an der ersten Auswahl empfangenen Reflexionen (vs) synthetisiert wird;
wobei
- der erste Bereich (140b) für jede jeweilige Kreuzungspunktposition (130) eine erste Überlappung mit den ersten Bereichen (142b) von mehr als einer der Mehrzahl von Kreuzungspunktpositionen (132) aufweist, wobei zumindest ein Teil der ersten Positionen der ersten Bereiche (142b) der mehr als einen der Mehrzahl von Kreuzungspunktpositionen (132) zumindest teilweise zwischen den ersten Positionen in dem ersten Bereich (140b) für die jeweilige Kreuzungspunktposition (130) eingestreut liegt, und
- der zweite Bereich (140a) oder die zweiten Bereiche für die jeweilige Kreuzungspunktposition (130) eine zweite Überlappung mit den zweiten Bereichen (142a) von mehr als einer der Mehrzahl von Kreuzungspunktpositionen (132) aufweist bzw. aufweisen, wobei zumindest ein Teil der zweiten Positionen des zweiten Bereichs (142a) der mehr als einen der Mehrzahl von Kreuzungspunktpositionen (132) zumindest teilweise zwischen den zweiten Positionen in dem zweiten Bereich (140a) für die jeweilige Kreuzungspunktposition (130) eingestreut liegt.

2. Verfahren nach Anspruch 1, wobei sich der erste Bereich (140b, 142b) für die jeweilige Kreuzungspunktposition (130) zumindest bis zu dem Versatz der nächstgelegenen Kreuzungspunktposition (132) von der jeweiligen Kreuzungspunktposition (130) erstreckt und/oder sich der zweite Bereich (54, 140a, 142a) für die jeweilige Kreuzungspunktposition (130) zumindest bis zu der nächstgelegenen Zeile der jeweiligen Kreuzungspunktposition (130) erstreckt.

3. Verfahren nach einem der vorhergehenden Ansprüche, wobei die erste Auswahl und die zweite Auswahl aperiodische Auswahlen sind.

4. Verfahren nach Anspruch 3, wobei Werte von Abständen (r(m)) zwischen aufeinanderfolgenden der ersten Abtastpositionen und/oder zwischen aufeinanderfolgenden der zweiten Abtastpositionen über einen vorbestimmten Abstandswertebereich verteilt liegen, der eine Mehrzahl von Abstandswerten enthält.

5. Verfahren nach Anspruch 4, wobei eine Untergrenze des vorbestimmten Abstandswertebereichs höchstens die Hälfte eines Durchschnitts der Werte der Abstände beträgt.

6. Verfahren nach einem der vorhergehenden Ansprüche, wobei die Kreuzungspunkte (j, 50, 80, 130, 132) auf einem zweidimensionalen periodischen Gitter von Positionen mit einer Periode angeordnet sind, die kleiner als der erste (140b, 142b) und der zweite (54, 140a, 142a) Bereich ist.

7. Verfahren nach einem der vorhergehenden Ansprüche, wobei durchschnittliche Unterabtastverhältnisse der ersten und der zweiten Auswahl höchstens die Hälfte einer Dichte der Kreuzungspunkte (j, 50, 80, 130, 132) entlang der Spalte (120) und/oder der Zeile (110) der jeweiligen Kreuzungspunktposition betragen.

8. Verfahren nach einem der vorhergehenden Ansprüche, wobei das Summieren der kreuzungspunktbasierten Bildwerte (Im(j, r)) für die Position (r) ein gewichtetes Summieren (w(j)) ist.

9. Verfahren nach einem der vorhergehenden Ansprüche, wobei das gewichtete Summieren (w(j)) ein Fenster von Kreuzungspunktpositionen (j, 50, 80, 130, 132) definiert, deren erste (140b, 142b) und/oder zweite (54, 140a, 142a) Bereiche sich sämtlich mit dem ersten (140b, 142b) und/oder zweiten (54, 140a, 142a) Bereich zumindest einer der Kreuzungspunktpositionen in dem Fenster überlappen.

10. Computerprogrammprodukt, umfassend ein Programm aus Anweisungen für ein programmierbares Verarbeitungssystem (40), die bei Ausführung durch das Verarbeitungssystem (40) des Ultraschallbildgebungssystems nach einem der Ansprüche 11 bis 15 das Verarbeitungssystem (40) veranlassen, das Verfahren nach einem der vorhergehenden Ansprüche durchzuführen.

11. Ultraschallbildgebungssystem, umfassend
- ein Array (10) aus Zeilen (110) und Spalten (120) von Ultraschallwandlern (100), die eine Erfassungsfläche definieren;
- eine Anregungsschaltung (12, 16, 44), die dazu konfiguriert ist, eine Ultraschallemission von auswählbaren ersten Ultraschallwandlern (100) in dem Array (10) zu bewirken;
- eine Empfangsschaltung (12, 14, 45), die dazu konfiguriert ist, Ultraschallmesssignale (vs) von auswählbaren zweiten Ultraschallwandlern (100) in dem Array (10) zu empfangen;
- ein Verarbeitungssystem (40), das konfiguriert ist zum
- Berechnen von Objektbildwerten für jede Position (r) in einem Bild der Ultraschallreflektivität (R, R(r)) in dem Objektraum durch Summieren kreuzungspunktbasierter Bildwerte (Im(j, r)) für die Position (r), die für eine Mehrzahl von Kreuzungspunktpositionen (j, 50, 80, 130, 132) bestimmt werden, die jeweils einer jeweiligen Kombination (j) aus einer Zeile und einer Spalte in dem Array (10) zugeordnet sind;
wobei das Verarbeitungssystem (40) dazu konfiguriert ist, den kreuzungspunktbasierten Bildwert (Im(j, r)) für jede jeweilige Kreuzungspunktposition (j, 50, 80, 130, 132) der Mehrzahl von Kreuzungspunktpositionen zu bestimmen durch
• Veranlassen der Ultraschallwandler (100), Ultraschallübertragungssignale (e0) von einer unterabgetasteten ersten Auswahl erster Abtastpositionen zu senden, die nicht weiter von der Spalte (120) der jeweiligen Kreuzungspunktposition entfernt liegen als von der Zeile (110) der jeweiligen Kreuzungspunktposition, wobei sich die erste Auswahl über einen ersten Bereich (140b, 142b) von Versätzen von der Zeile der jeweiligen Kreuzungspunktposition erstreckt;
• Empfangen von Reflexionen (vs) der Ultraschallübertragungssignale (e0) aus dem Objektraum an einer unterabgetasteten zweiten Auswahl zweiter Abtastpositionen entlang der Zeile (24, 110, 52) der jeweiligen Kreuzungspunktposition oder an zweiten Auswahlen zweiter Abtastpositionen in einer unterabgetasteten dritten Auswahl von Zeilen (84), die die Zeile der jeweiligen Kreuzungspunktposition einschließt, wobei sich die zweite Auswahl oder die zweiten Auswahlen über einen zweiten Bereich (54, 140a, 142a) der zweiten Positionen entlang der Zeile der jeweiligen Kreuzungspunktposition erstrecken und zumindest ein Teil der Reflexionen (vs) in der zweiten Auswahl oder den zweiten Auswahlen gleichzeitig empfangen wird;
• Berechnen, für die jeweilige Kreuzungspunktposition (j, 50, 80, 130, 132), des kreuzungspunktbasierten Bildwertes (Im(j, r)), der der Reflektivität (R, R(r)) an der Position (r) in dem Objektraum zugeordnet ist und aus den an der zweiten Auswahl oder den zweiten Auswahlen als Reaktion auf die Ultraschallübertragungssignale (e0) an der ersten Auswahl empfangenen Reflexionen (vs) synthetisiert wird;
wobei
- der erste Bereich (140b) für jede jeweilige Kreuzungspunktposition (130) eine erste Überlappung mit den ersten Bereichen (142b) von mehr als einer der Mehrzahl von Kreuzungspunktpositionen (132) aufweist, wobei zumindest ein Teil der ersten Positionen der ersten Bereiche (142b) der mehr als einen der Mehrzahl von Kreuzungspunktpositionen (132) zumindest teilweise zwischen den ersten Positionen in dem ersten Bereich (140b) für die jeweilige Kreuzungspunktposition (130) eingestreut liegt, und
- der zweite Bereich (140a) oder die zweiten Bereiche für die jeweilige Kreuzungspunktposition (130) eine zweite Überlappung mit den zweiten Bereichen (142a) von mehr als einer der Mehrzahl von Kreuzungspunktpositionen (132) aufweist bzw. aufweisen, wobei zumindest ein Teil der zweiten Positionen des zweiten Bereichs (142a) der mehr als einen der Mehrzahl von Kreuzungspunktpositionen (132) zumindest teilweise zwischen den zweiten Positionen in dem zweiten Bereich (140a) für die jeweilige Kreuzungspunktposition (130) eingestreut liegt.

12. Ultraschallbildgebungssystem (42) nach Anspruch 11, wobei
- die Anregungsschaltung (12, 16, 44) einen Signalgenerator (44) und eine erste Zugriffsschaltung (16) umfasst, die zwischen den Signalgenerator (44) und Spalten (120) des Arrays (10) gekoppelt ist, um Ultraschall mit einer durch den Signalgenerator (44) definierten Zeitabhängigkeit von den ersten auswählbaren Positionen in dem Array (10) zu erzeugen, wobei die erste Zugriffsschaltung (16) dazu konfiguriert ist, Positionen der ersten Ultraschallwandler (100) unter Steuerung durch die Verarbeitungsschaltung (40) auszuwählen;
- die Empfangsschaltung (12, 14, 45) eine Detektionsschaltung (45) und eine zweite Zugriffsschaltung (14) umfasst, die zwischen Spalten (120) des Arrays (10) und die Detektionsschaltung (45) gekoppelt ist, wobei die Detektionsschaltung (45) dazu konfiguriert ist, eine Zeitabhängigkeit der Ultraschallmessungen (vs) von den auswählbaren zweiten Ultraschallwandlern (100) in dem Array (10) zu detektieren, wobei die zweite Zugriffsschaltung (14) dazu konfiguriert ist, Positionen der zweiten Ultraschallwandler (100) unter Steuerung durch die Verarbeitungsschaltung (40) auszuwählen.

13. Ultraschallbildgebungssystem (42) nach Anspruch 11 oder 12, wobei sich der erste (140b, 142b) und/oder der zweite (54, 140a, 142a) Bereich für die jeweilige Kreuzungspunktposition (130) zumindest bis zu den nächstgelegenen Kreuzungspunktpositionen (132) von der jeweiligen Kreuzungspunktposition (130) entlang der Spalte (120) bzw. der Zeile (110) der jeweiligen Kreuzungspunktposition erstreckt bzw. erstrecken.

14. Ultraschallbildgebungssystem (42) nach einem der Ansprüche 11-13, wobei die erste Auswahl und die zweite Auswahl aperiodische Auswahlen sind.

15. Ultraschallbildgebungssystem (42) nach einem der Ansprüche 11-14, wobei das Summieren der kreuzungspunktbasierten Bildwerte (Im(j, r)) für die Position (r) ein gewichtetes Summieren (w(j)) ist.

## Revendications

1. Procédé de formation d'une image d'un objet par réflectivité ultrasonore dans un espace objet, utilisant un réseau (10) de rangées (110) et de colonnes (120) de transducteurs ultrasonores (100) qui définissent une surface de détection adjacente à l'espace objet, le procédé comprenant :
- le calcul de valeurs d'image d'objet pour chaque position (r) dans l'image de réflectivité ultrasonore (R, R(r)) dans l'espace objet en sommant des valeurs d'image basées sur des points de croisement (Im(j, r)) pour la position (r) déterminée pour une pluralité d'emplacements de points de croisement (j, 50, 80, 130, 132) qui sont chacun associés à une combinaison respective (j) d'une rangée et d'une colonne dans le réseau (10) ;
dans lequel la valeur d'image basée sur le point de croisement (Im(j, r)) pour chaque emplacement de point de croisement respectif (j, 50, 80, 130, 132) de la pluralité d'emplacements de points de croisement est déterminée par :
• l'émission de signaux de transmission ultrasonore (e0) à partir des transducteurs ultrasonores (100) à une première sélection sous-échantillonnée de premiers emplacements d'échantillonnage ne se trouvant pas plus loin de la colonne (120) de l'emplacement de point de croisement (130) respectif que de la rangée (110) de l'emplacement de point de croisement (130) respectif, la première sélection s'étendant sur une première plage (140b, 142b) de décalages à partir de la rangée des premiers emplacements d'échantillonnage ;
• la réception de réflexions (vs) des signaux de transmission ultrasonore (e0) provenant de l'espace objet à une deuxième sélection sous-échantillonnée de deuxièmes emplacements d'échantillonnage le long de la rangée (24, 110, 52) de l'emplacement de point de croisement respectif, ou à des deuxièmes sélections de deuxièmes emplacements d'échantillonnage à une troisième sélection sous-échantillonnée de rangées (84) qui comprend la rangée de l'emplacement de point de croisement (130) respectif, la deuxième sélection ou les deuxièmes sélections s'étendant sur une deuxième plage (54, 140a, 142a) des deuxièmes emplacements le long de la rangée de l'emplacement de point de croisement respectif, au moins une partie des réflexions (vs) dans la deuxième sélection ou les deuxièmes sélections étant reçue simultanément ;
• le calcul, pour l'emplacement de point de croisement respectif (j, 50, 80, 130, 132), de la valeur d'image basée sur le point de croisement (Im (j, r)) associée à la réflectivité (R, R(r)) à la position (r) dans l'espace objet, synthétisée à partir des réflexions (vs) reçues à la deuxième sélection ou aux deuxièmes sélections en réponse aux signaux de transmission ultrasonore (e0) à la première sélection ;
dans lequel
- la première plage (140b) pour chaque emplacement de point de croisement (130) respectif présente un premier chevauchement avec les premières plages (142b) de plus d'un des emplacements de points de croisement de la pluralité (132), au moins une partie des premiers emplacements des premières plages (142b) de plus d'un des emplacements de points de croisement de la pluralité (132) étant au moins partiellement intercalée entre les premiers emplacements de la première plage (140b) pour l'emplacement de point de croisement (130) respectif, et
- la deuxième plage (140a) ou les deuxièmes plages pour l'emplacement de point de croisement (130) respectif présente un deuxième chevauchement avec les deuxièmes plages (142a) de plus d'un des emplacements de points de croisement de la pluralité (132), au moins une partie des deuxièmes emplacements de la deuxième plage (142a) de plus d'un des emplacements de points de croisement de la pluralité (132) étant au moins partiellement intercalée entre les deuxièmes emplacements de la deuxième plage (140a) pour l'emplacement de point de croisement (130) respectif.

2. Procédé selon la revendication 1, dans lequel la première plage (140b, 142b) pour l'emplacement de point de croisement (130) respectif s'étend au moins jusqu'au décalage de l'emplacement de point de croisement (132) le plus proche à partir de l'emplacement de point de croisement (130) respectif et/ou la deuxième plage (54, 140a, 142a) pour l'emplacement de point de croisement (130) respectif s'étend au moins jusqu'à la rangée la plus proche de l'emplacement de point de croisement (130) respectif.

3. Procédé selon l'une quelconque des revendications précédentes, dans lequel la première sélection et la deuxième sélection sont des sélections apériodiques.

4. Procédé selon la revendication 3, dans lequel les valeurs des distances (r(m)) entre des premiers emplacements d'échantillonnage successifs et/ou entre des deuxièmes emplacements d'échantillonnage successifs sont réparties sur une plage de valeurs de distance prédéterminée qui contient une pluralité de valeurs de distance.

5. Procédé selon la revendication 4, dans lequel une limite inférieure de la plage de valeurs de distance prédéterminée est au plus égale à la moitié d'une moyenne des valeurs des distances.

6. Procédé selon l'une quelconque des revendications précédentes, dans lequel les points de croisement (j, 50, 80, 130, 132) sont situés sur une grille bidimensionnelle périodique d'emplacements, avec une période inférieure aux premières (140b, 142b) et deuxièmes (54, 140a, 142a) plages.

7. Procédé selon l'une quelconque des revendications précédentes, dans lequel les rapports moyens de sous-échantillonnage des première et deuxième sélections sont au plus égales à la moitié d'une densité des points de croisement (j, 50, 80, 130, 132) le long de la colonne (120) et/ou de la rangée (110) de l'emplacement de point de croisement respectif.

8. Procédé selon l'une quelconque des revendications précédentes, dans lequel ladite sommation des valeurs d'image basées sur les points de croisement (Im(j, r)) pour la position (r) est une sommation pondérée (w(j)).

9. Procédé selon l'une quelconque des revendications précédentes, dans lequel la sommation pondérée (w(j)) définit une fenêtre d'emplacements de points de croisement (j, 50, 80, 130, 132) dont les premières (140b, 142b) et/ou deuxièmes (54, 140a, 142a) plages se chevauchent toutes avec la première (140b, 142b) et/ou la deuxième (54, 140a, 142a) plage d'au moins un des emplacements de points de croisement dans la fenêtre.

10. Produit logiciel informatique, comprenant un programme d'instructions pour un système de traitement programmable (40) qui, lorsqu'il est exécuté par le système de traitement (40) du système d'imagerie ultrasonore selon l'une quelconque des revendications 11 à 15, amène le système de traitement (40) à exécuter le procédé selon l'une quelconque des revendications précédentes.

11. Système de formation d'image de réflectivité ultrasonore, comprenant :
- un réseau (10) de rangées (110) et de colonnes (120) de transducteurs ultrasonores (100) qui définissent une surface de détection ;
- un circuit d'excitation (12, 16, 44) configuré pour provoquer l'émission d'ultrasons à partir de premiers transducteurs ultrasonores (100) sélectionnables dans le réseau (10) ;
- un circuit de réception (12, 14, 45) configuré pour recevoir des signaux de mesure ultrasonore (vs) provenant de deuxièmes transducteurs ultrasonores (100) sélectionnables dans le réseau (10) ;
- un système de traitement (40) configuré pour
- calculer des valeurs d'image d'objet pour chaque position (r) dans une image de réflectivité ultrasonore (R, R(r)) dans l'espace objet en sommant des valeurs d'image basées sur des points de croisement (Im(j, r)) pour la position (r) déterminée pour une pluralité d'emplacements de points de croisement (j, 50, 80, 130, 132) qui sont chacun associés à une combinaison respective (j) d'une rangée et d'une colonne dans le réseau (10) ;
dans lequel le système de traitement (40) est configuré pour déterminer la valeur d'image basée sur le point de croisement (Im(j, r)) pour chaque emplacement de point de croisement respectif (j, 50, 80, 130, 132) de la pluralité d'emplacements de points de croisement en
• provoquant l'émission de signaux de transmission ultrasonore (e0) par les transducteurs ultrasonores (100) à partir d'une première sélection sous-échantillonnée de premiers emplacements d'échantillonnage qui ne se trouvent pas plus loin de la colonne (120) de l'emplacement de point de croisement respectif que de la rangée (110) de l'emplacement de point de croisement respectif, la première sélection s'étendant sur une première plage (140b, 142b) de décalages à partir de la rangée de l'emplacement de point de croisement respectif ;
• recevant des réflexions (vs) des signaux de transmission ultrasonore (e0) provenant de l'espace objet à une deuxième sélection sous-échantillonnée de deuxièmes emplacements d'échantillonnage le long de la rangée (24, 110, 52) de l'emplacement de point de croisement respectif, ou à des deuxièmes sélections de deuxièmes emplacements d'échantillonnage à une troisième sélection sous-échantillonnée de rangées (84) qui comprend la rangée de l'emplacement de point de croisement respectif, la deuxième sélection ou les deuxièmes sélections s'étendant sur une deuxième plage (54, 140a, 142a) des deuxièmes emplacements le long de la rangée de l'emplacement de point de croisement respectif ; au moins une partie des réflexions (vs) dans la deuxième sélection ou les deuxièmes sélections étant reçue simultanément ;
• calculant, pour l'emplacement de point de croisement respectif (j, 50, 80, 130, 132), la valeur d'image basée sur le point de croisement (Im(j, r)) associée à la réflectivité (R, R(r)) à la position (r) dans l'espace objet, synthétisée à partir des réflexions (vs) reçues à la deuxième sélection ou aux deuxièmes sélections en réponse aux signaux de transmission ultrasonore (e0) à la première sélection ;
dans lequel
- la première plage (140b) pour chaque emplacement de point de croisement (130) respectif présente un premier chevauchement avec les premières plages (142b) de plus d'un des emplacements de points de croisement de la pluralité (132), au moins une partie des premiers emplacements des premières plages (142b) de plus d'un des emplacements de points de croisement de la pluralité (132) se trouvant au moins partiellement intercalée entre les premiers emplacements dans la première plage (140b) pour l'emplacement de point de croisement (130) respectif, et
- la deuxième plage (140a) ou les deuxièmes plages pour l'emplacement de point de croisement (130) respectif présente un deuxième chevauchement avec les deuxièmes plages (142a) de plus d'un des emplacements de points de croisement de la pluralité (132), au moins une partie des deuxièmes emplacements dans la deuxième plage (142a) de plus d'un des emplacements de points de croisement de la pluralité (132) se trouvant au moins partiellement intercalée entre les deuxièmes emplacements dans la deuxième plage (140a) pour l'emplacement de point de croisement (130) respectif.

12. Système de formation d'image de réflectivité ultrasonore (42) selon la revendication 11, dans lequel
- le circuit d'excitation (12, 16, 44) comprend un générateur de signaux (44) et un premier circuit d'accès (16) couplé entre le générateur de signaux (44) et les colonnes (120) du réseau (10), pour générer des ultrasons avec une dépendance temporelle définie par le générateur de signaux (44) à partir des premiers emplacements sélectionnables pour le réseau (10), le premier circuit d'accès (16) étant configuré pour sélectionner des emplacements dans des premiers transducteurs ultrasonores (100) sous le contrôle du circuit de traitement (40) ;
- le circuit de réception (12, 14, 45) comprend un circuit de détection (45) et un deuxième circuit d'accès (14) couplé entre les colonnes (120) du réseau (10) et le circuit de détection (45), le circuit de détection (45) étant configuré pour détecter une dépendance temporelle des mesures ultrasonores (vs) provenant des deuxièmes transducteurs ultrasonores (100) sélectionnables dans le réseau (10), le deuxième circuit d'accès (14) étant configuré pour sélectionner des emplacements des deuxièmes transducteurs ultrasonores (100) sous le contrôle du circuit de traitement (40).

13. Système de formation d'image de réflectivité ultrasonore (42) selon la revendication 11 ou la revendication 12, dans lequel les premières (140b, 142b) et/ou deuxièmes (54, 140a, 142a) plages pour l'emplacement de point de croisement (130) respectif s'étendent au moins jusqu'aux emplacements de points de croisement (132) les plus proches à partir de l'emplacement de point de croisement (130) respectif le long de la colonne (120) et/ou de la rangée (110) de l'emplacement de point de croisement respectif, respectivement.

14. Système de formation d'image de réflectivité ultrasonore (42) selon l'une quelconque des revendications 11 à 13, dans lequel la première sélection et la deuxième sélection sont des sélections apériodiques.

15. Système de formation d'image de réflectivité ultrasonore (42) selon l'une quelconque des revendications 11 à 14, dans lequel ladite sommation des valeurs d'image basées sur les points de croisement (Im(j, r)) pour la position (r) est une sommation pondérée (w(j)).
